# EUROPEAN PATENT SPECIFICATION

(11) **EP 3 973 936 B1**
(45) Date of publication and mention of the grant of the patent: **05.07.2023**
(21) Application number: 21197150.2
(22) Date of filing: 16.09.2021
(51) Int. Cl.: A61F 13/49, A61F 13/494, A61F 13/496, A61F 13/53

(54) **UNDERPANTS-SHAPED ABSORBENT ARTICLE**
UNTERHOSENFÖRMIGER ABSORBIERENDER ARTIKEL
ARTICLE ABSORBANT EN FORME DE CULOTTE

(30) Priority: 29.09.2020 JP 2020164147
(43) Date of publication of application: 30.03.2022
(73) Proprietor: Unicharm Corporation, Shikokuchuo-shi, Ehime 799-0111 (JP)
(72) Inventor: MATSUO, Takanori, Kanonji-shi, 769-1602 (JP); FUJII, Keishi, Kanonji-shi, 769-1602 (JP)
(74) Representative: Dolleymores

(56) References cited:
- WO-A1-2018/096877
- JP-A- 2015 116 471
- US-A1- 2016 206 483
- US-A1- 2018 168 888

## Description

### [Technical Field]

The present invention relates to an underpants-shaped absorbent article.

### [Background Art]

A disposable diaper is known as an example of an absorbent article. Patent Document 1 discloses a disposable diaper in which an absorbent layer (absorbent body) is provided between a liquid-permeable upper-face sheet and a liquid-impermeable back-face sheet, and a front panel and a back panel are provided on the front and back sides of the absorbent layer in the longitudinal direction. At least one groove that extends in the longitudinal direction is provided in the widthwise central portion of the upper face of the absorbent layer of this diaper, and excreted fluid that has permeated in the thickness direction in the widthwise central portion is immediately guided forward and backward along the groove in the longitudinal direction and can be absorbed by the front and back portions of the absorbent layer.

### [Citation List]

### [Patent Literature]

[Patent Document 1] Japanese Patent Publication No. 4092319

US 2016/0206483 A1 relates to a disposable absorbent pant having a belt structure with front and rear belt portions and a longitudinally channeled absorbent core structure.

US 2018/0168888 A1 discloses an absorbent article including an absorbent chassis comprising an absorbent core having one or more channels.

JP 2015-116471 A relates to an absorbent article having an absorber provided with bending guidance/induction parts which may consist of grooves/channels.

### [Summary of Invention]

### [Technical Problem]

The groove in the absorbent layer disclosed in Patent Document 1 disperses excreted fluid over a wide area, and also serves as a folding point for allowing deformation of the absorbent body. If the groove is formed so as to extend up to the waist region of the user, the absorbent body may bend at the groove due to movement during usage, such as the user closing their legs, and the deformation caused by the bending may reach a longitudinal end portion of the absorbent layer (absorbent body) and allow leakage from the end portion of the absorbent body.

The present invention was achieved in light of conventional problems such as that described above, and an aspect of the present invention is to provide an underpants-shaped absorbent article that induces deformation of the absorbent body while also suppressing leakage from a longitudinal end portion of the absorbent body.

### [Solution to Problem]

A main aspect of the present invention for achieving the above-described aspect is An underpants-shaped absorbent article having a longitudinal direction, a width direction, and a thickness direction that intersect each other in an unfolded state, the underpants-shaped absorbent article including: a front waist portion; a back waist portion; an absorbent main body that includes an absorbent body, a first joining portion in which a non-skin-side surface of an end portion of the absorbent main body on one side in the longitudinal direction is joined to the front waist portion using an adhesive; and a second joining portion in which a non-skin-side surface of an end portion of the absorbent main body on another side in the longitudinal direction is joined to the back waist portion using an adhesive, the absorbent main body being folded one time at a central position in the longitudinal direction, widthwise end portions of the front waist portion and widthwise end portions of the back waist portion being joined in a pair of waist joining portions, the absorbent body including a low-basis-weight portion in at least a central portion in the longitudinal direction, the low-basis-weight portion extending along the longitudinal direction, the low-basis-weight portion having a smaller basis weight than a surrounding area, a high-stiffness portion being provided in at least one of a longitudinal region extending from an inward end of the first joining portion to an outward end of the low-basis-weight portion and a longitudinal region extending from an inward end of the second joining portion to another outward end of the low-basis-weight portion, the high-stiffness portion being a portion that has a higher stiffness than the central position. Features of the present invention other than the above will become clear by reading the description of the present specification with reference to the accompanying drawings.

### [Advantageous Effects of Invention]

According to the present invention, it is possible to provide an underpants-shaped absorbent article that induces deformation of the absorbent body while also suppressing leakage from a longitudinal end portion of the absorbent body.

### [Brief Description of the Drawings]

FIG. 1 is a schematic perspective view of a pull-on disposable diaper 1 (diaper 1) .
FIG. 2 is a schematic plan view of the diaper 1 in an unfolded and stretched state.
FIG. 3 is a plan view illustrating a configuration of an absorbent main body 10 and leak-proof wall portions 50.
FIG. 4 is a schematic cross-sectional view taken along a line A-A of FIG. 3.
FIG. 5 is a schematic plan view of virtual lines H on the front side and the back side of the diaper 1.
FIG. 6 is a plan view showing only the front side of an absorbent body 11 when the diaper 1 has been folded at a central position FL in the longitudinal direction.

### [Description of Embodiments]

At least the following matters will become clear with the description of this specification and the attached drawings.

An underpants-shaped absorbent article having a longitudinal direction, a width direction, and a thickness direction that intersect each other in an unfolded state, the underpants-shaped absorbent article including: a front waist portion; a back waist portion; an absorbent main body that includes an absorbent body, a first joining portion in which a non-skin-side surface of an end portion of the absorbent main body on one side in the longitudinal direction is joined to the front waist portion using an adhesive; and a second joining portion in which a non-skin-side surface of an end portion of the absorbent main body on another side in the longitudinal direction is joined to the back waist portion using an adhesive, the absorbent main body being folded one time at a central position in the longitudinal direction, widthwise end portions of the front waist portion and widthwise end portions of the back waist portion being joined in a pair of waist joining portions, the absorbent body including a low-basis-weight portion in at least a central portion in the longitudinal direction, the low-basis-weight portion extending along the longitudinal direction, the low-basis-weight portion having a smaller basis weight than a surrounding area, a high-stiffness portion being provided in at least one of a longitudinal region extending from an inward end of the first joining portion to an outward end of the low-basis-weight portion and a longitudinal region extending from an inward end of the second joining portion to another outward end of the low-basis-weight portion, the high-stiffness portion being a portion that has a higher stiffness than the central position.

According to such an underpants-shaped absorbent article, the low-basis-weight portion is arranged in the central portion in the longitudinal direction, which corresponds to the crotch region of the wearer. This makes it possible to induce deformation of the absorbent body from the low-basis-weight portion so as to fit to the crotch region. Also, excreted urine that has moved through the low-basis-weight portion is dispersed throughout the absorbent body, and the high-stiffness portion can suppress deformation of the absorbent body because the high-stiffness portion is located outside the longitudinal outward end of the low-basis-weight portion. Preventing deformation from occurring up to the longitudinal end portion of the absorbent main body makes it possible to suppress the occurrence of leakage.

In such an underpants-shaped absorbent article, it is desirable
that the high-stiffness portion includes a compressed portion, and
that in a case where a virtual line is drawn such that a position of a longitudinal inward end of each widthwise end of either of the first joining portion and the second joining portion is connected to a widthwise center point located at the central position in the longitudinal direction,
   the low-basis-weight portion extends in the longitudinal direction, to a position identical to the virtual line or outward in the longitudinal direction beyond the virtual line, and
that the compressed portion is provided in a portion that is overlapped with a longitudinal end portion of the low-basis-weight portion in a view in the thickness direction.

According to this underpants-shaped absorbent article, the virtual line is a line that can be formed by the weight of the absorbent body that has absorbed urine after urination. After urination, a fold line is likely to be formed in the absorbent main body along the virtual line. If the low-basis-weight portion ends at a position below the virtual line (inward thereof in the longitudinal direction), there would be a risk that the dispersion of excreted fluid by the low-basis-weight portion would be stopped by creases formed along the virtual line. However, if the low-basis-weight portion extends in the longitudinal direction to the position identical to the virtual line or outward beyond the virtual line (upward thereof), the dispersion of excreted fluid is less likely to be stopped. Also, because the compressed portion is located in a portion overlapped with the end portion of the low-basis-weight portion, the liquid lifting capability is improved at the end portion of the low-basis-weight portion, and the excreted fluid transmitted by the low-basis-weight portion can be further dispersed in the compressed portion.

In such an underpants-shaped absorbent article, it is desirable
that the high-stiffness portion includes a compressed portion, and
that a non-compressed region is provided in a predetermined range that includes the central position in the longitudinal direction,
   the non-compressed region being a region that does not include the compressed portion.

According to this underpants-shaped absorbent article, the non-compressed region is a portion that comes into contact with the wearer's crotch, and due to not providing the compressed portion in the crotch portion, it is possible to further improve the ability to immediately absorb excreted urine and to swiftly disperse the urine in the longitudinal direction with use of the low-basis-weight portion. Also, by providing the non-compressed region at the crotch, there is no portion having high stiffness, and therefore the diaper can more easily fit to the crotch region when the wearer closes their legs, for example.

In such an underpants-shaped absorbent article, it is desirable that
the high-stiffness portion is provided in at least part of a portion of the absorbent body that is overlapped with either of the first joining portion and the second joining portion in a view in the thickness direction.

According to this underpants-shaped absorbent article, the portion of the absorbent body that is overlapped with either of the first joining portion and the second joining portion in a view in the thickness direction is also overlapped with the adhesive in the joining portions. Because this portion has a high-stiffness portion, the stiffness is even higher than that of the portion that is not overlapped with either of the first joining portion and the second joining portion. In other words, deformation at the low-basis-weight portion is suppressed by the high-stiffness portion in the region inward of the joining portions in the longitudinal direction, and furthermore, deformation can be more reliably suppressed by the portion where the high-stiffness portion is overlapped with either of the first joining portion and the second joining portion in a view in the thickness direction. Therefore, deformation can be suppressed neatly without forming unnatural folds, wrinkles, or the like due to rapid suppression.

In such an underpants-shaped absorbent article, it is desirable
that the adhesive in the first joining portion and the second joining portion is provided extending along the longitudinal direction, at intermittent locations in the width direction, and
that the high-stiffness portion is provided in at least part of a portion of the absorbent body that is overlapped with either of the first joining portion and the second joining portion in a view in the thickness direction.

According to this underpants-shaped absorbent article, the adhesive is provided intermittently, thus making it possible to prevent the joining portions from becoming too hard. The portion of the absorbent body where the adhesive and the high-stiffness portion are overlapped in a view in the thickness direction has an even higher stiffness, and therefore deformation of the low-basis-weight portion can be more reliably suppressed. Also, by providing the adhesive intermittently in the width direction, it is possible to prevent the entirety of the joining portions from becoming excessively hard even if they are overlapped with the high-stiffness portion, thus making it unlikely for the wearer to feel uncomfortable.

In such an underpants-shaped absorbent article, it is desirable
that the high-stiffness portion includes a compressed portion, and
that an overlap region and a non-overlap region are provided with respect to the longitudinal direction,
   the overlap region being a region that includes the compressed portion being overlapped with the low-basis-weight portion,
   the non-overlap region being a region that includes the compressed portion and that is located outward of the overlap region, and
that a longitudinal length of the overlap region is less than or equal to a longitudinal length of the non-overlap region.

According to this underpants-shaped absorbent article, the overlap region is a region where excreted fluid that has moved along the low-basis-weight portion is passed to the non-overlap region. The non-overlap region is a region where the effect of lifting excreted fluid is improved by the compressed portion, and where excreted fluid can be dispersed over a wider area. If the overlap region does not exist, it may be difficult for excreted fluid to be properly passed to the non-overlap region, and therefore it is necessary to provide the overlap region with at least a minimum required size. Accordingly, if the overlap region is provided such that the longitudinal length of the overlap region is less than or equal to the longitudinal length of the non-overlap region, the smaller overlap region increases the possibility that liquid is smoothly moved by the low-basis-weight portion and dispersed by the non-overlap region, compared with the case where the overlap region is provided such that the longitudinal length of the overlap region is longer than the longitudinal length of the non-overlap region.

In such an underpants-shaped absorbent article, it is desirable
that the high-stiffness portion includes a compressed portion,
that the underpants-shaped absorbent article has a vertical direction,
that in the vertical direction, in a state where the absorbent main body has been folded at the central position,
   a side on which the front waist portion and the back waist portion are arranged is an upper side, and
   a side corresponding to the central position is a lower side,
that an overlap region is provided with respect to the vertical direction,
   the overlap region being a region that includes the compressed portion being overlapped with the low-basis-weight portion, and
that letting a low-basis-weight-portion central region be a region of the low-basis-weight portion that extends in the vertical direction from a lower end of the overlap region to the central position,
   a vertical length of the low-basis-weight-portion central region is longer than a vertical length of the overlap region.

According to this underpants-shaped absorbent article, the low-basis-weight-portion central region is a region that is close to the wearer's urination orifice, and if the overlap region is longer than the low-basis-weight-portion central region, the immediate absorption performance near the urination orifice deteriorates, and leakage may occur. For this reason, by setting the length of the low-basis-weight-portion central region longer than the length of the overlap region, excreted urine is rapidly moved in the longitudinal direction by the low-basis-weight portion, and excreted fluid is passed in the overlap region, and thus excreted fluid can be easily dispersed over a wide area.

In such an underpants-shaped absorbent article, it is desirable
that the high-stiffness portion includes a compressed portion,
that the underpants-shaped absorbent article has a vertical direction,
that in the vertical direction, in a state where the absorbent main body has been folded at the central position,
   a side on which the front waist portion and the back waist portion are arranged is an upper side, and
   a side corresponding to the central position is a lower side,
that an overlap region and a non-overlap region are provided with respect to the vertical direction,
   the overlap region being a region that includes the compressed portion being overlapped with the low-basis-weight portion,
   the non-overlap region being a region that includes the compressed portion and that is located upward of the overlap region, and
that letting a low-basis-weight-portion central region be a region of the low-basis-weight portion that extends in the vertical direction from a lower end of the overlap region to the central position,
   a vertical length of the non-overlap region is longer than a vertical length of the low-basis-weight-portion central region.

According to this underpants-shaped absorbent article, if the low-basis-weight-portion central region extends too far to the front side, the region that can absorb excreted fluid that has been moved and dispersed will be insufficiently large, and leakage may occur. Furthermore, if the low-basis-weight-portion central region extends too far to the back side, deformation of the absorbent main body will be promoted, and there is a risk that the stool storage space cannot be sufficiently secured and leakage will occur. Accordingly, by setting the non-overlap region longer while also providing the low-basis-weight-portion central region over an appropriate range, after a liquid has been rapidly dispersed by the low-basis-weight portion, it is possible to promote the dispersion of the liquid over a wider area by the liquid lifting effect of the non-overlap region that is provided over a wider area.

In such an underpants-shaped absorbent article, it is desirable that
the high-stiffness portion has either of
a portion in which a high-density portion is formed in a linear manner, and
a portion in which the high-density portion is dot-shaped and a plurality of the high-density portions are formed,
   the high-density portion being a portion whose density is higher than a surrounding area.

According to this underpants-shaped absorbent article, with the portion in which the high-density portion is formed in a linear manner, it is possible to disperse excreted fluid to a greater degree along the linear high-density portion. Also, it is possible to adjust the stiffness to any strength by using a linear pattern. With the portion in which the high-density portion is dot-shaped and a plurality of the high-density portions are formed, each dot-shaped high-density portion serves as a starting point for absorbing a liquid, and the excreted fluid can be dispersed by spreading out from each dot.

In such an underpants-shaped absorbent article, it is desirable
that the underpants-shaped absorbent article has a vertical direction,
that in the vertical direction, in a state where the absorbent main body has been folded at the central position,
   a side on which the front waist portion and the back waist portion are arranged is an upper side, and
   a side corresponding to the central position is a lower side, and
that letting a side on which the front waist portion is located be a front side, and
that letting a side on which the back waist portion is located be a back side,
   a vertical length of the low-basis-weight portion on the front side is longer than a vertical length of the low-basis-weight portion on the back side.

According to this underpants-shaped absorbent article, a longer length is set for the low-basis-weight portion on the front side near the urination orifice, thus making it possible to improve the dispersion of excreted fluid. On the other hand, if the low-basis-weight portion is too long on the back side, there is a risk that the low-basis-weight portion promotes deformation of the absorbent main body, and that it is not possible to secure a sufficient stool storage space. Therefore it is preferable that the length on the back side is set so as to not be too long .

In such an underpants-shaped absorbent article, it is desirable
that the high-stiffness portion includes a compressed portion,
that an overlap region and a non-overlap region are provided,
   the overlap region being a region that includes the compressed portion being overlapped with the low-basis-weight portion in the longitudinal direction,
   the non-overlap region being a region that includes the compressed portion and that is located outward of the overlap region in the longitudinal direction, and
that the non-overlap region extends up to either of a longitudinal end portion and a widthwise end portion of the absorbent body.

According to this underpants-shaped absorbent article, excreted fluid that has been moved in the longitudinal direction by the low-basis-weight portion can be widely dispersed up to either of the longitudinal end portion and the widthwise end portion of the absorbent body, and more efficient absorption can be realized.

In such an underpants-shaped absorbent article, it is desirable
that the underpants-shaped absorbent article further comprises a pair of leak-proof wall portions that are respectively provided on widthwise sides of the absorbent main body,
that the pair of leak-proof wall portions each include
   a base end portion that cannot rise in the thickness direction, and
   a rising portion that can rise from the base end portion to a skin side in the thickness direction due to an elastic member that can stretch and contract in the longitudinal direction,
that the high-stiffness portion includes a compressed portion, and
that in the width direction, the compressed portion is provided in an entirety of a region that extends from an inward end of one of the base end portions to an inward end of another one of the base end portions.

According to this underpants-shaped absorbent article, in order to block excreted urine, it is preferable that the region inward of the leak-proof wall portions in the width direction does not deform. If the compressed portion is provided between the base end portions of the leak-proof wall portions, such deformation is suppressed, and the leak prevention function can be further improved.

In such an underpants-shaped absorbent article, it is desirable that
the compressed portion is provided so as to cross at least a portion of the virtual line in a plan view.

According to this underpants-shaped absorbent article, the absorbent body is likely to fold along the virtual lines after urination, but by providing the compressed portion so as to cross the virtual lines, it is possible to suppress folding of the absorbent article after urination and to prevent the dispersion of excreted fluid from being stopped due to such folding.

In such an underpants-shaped absorbent article, it is desirable that
the absorbent body includes an absorbent core and a liquid-permeable core-wrapping sheet,
the core-wrapping sheet covering the absorbent core from a skin side and a non-skin side in the thickness direction, and in the low-basis-weight portion,
a portion of the core-wrapping sheet located on the skin side is joined to a portion of the core-wrapping sheet located on the non-skin side.

According to this underpants-shaped absorbent article, if the core-wrapping sheets on the skin side and the non-skin side are not joined to each other in the low-basis-weight portion, the absorbent body swells when urine is absorbed, and the interior constituent materials of the absorbent body may move, and there is a risk that the low-basis-weight portion will eliminate due to such factors. By joining the core-wrapping sheets on the skin side and the non-skin side to each other, it is possible to prevent the low-basis-weight portion from being eliminated, and the effects of movement and dispersion of excreted fluid by the low-basis-weight portion can be realized continuously over time.

### Embodiment

Hereinafter, embodiment of an underpants-shaped absorbent article according to the present invention will be described by way of example of a pull-on disposable diaper for infants. However, the underpants-shaped absorbent article according to the present invention is not limited to this, and can be applied to pull-on disposable diapers for adults, sanitary shorts, and the like.

### Configuration of pull-on disposable diaper 1

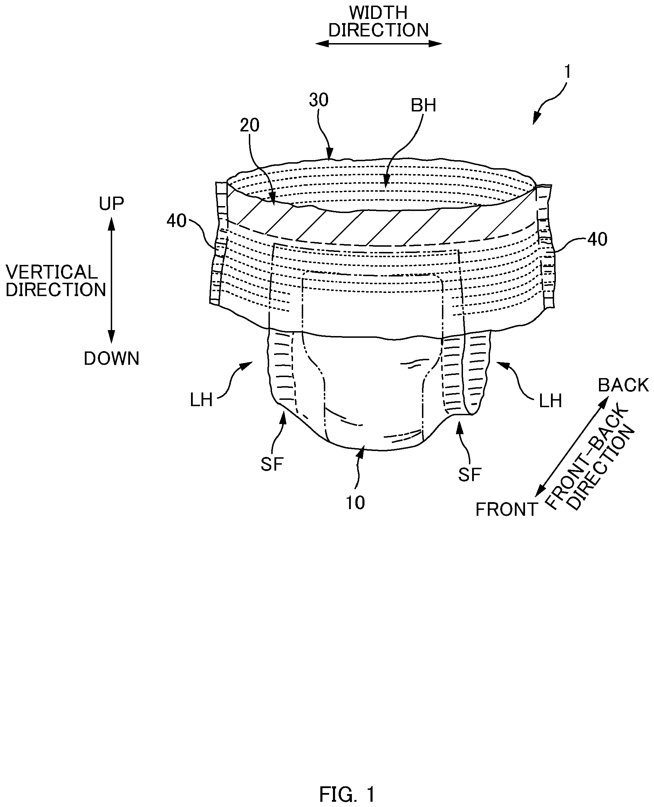

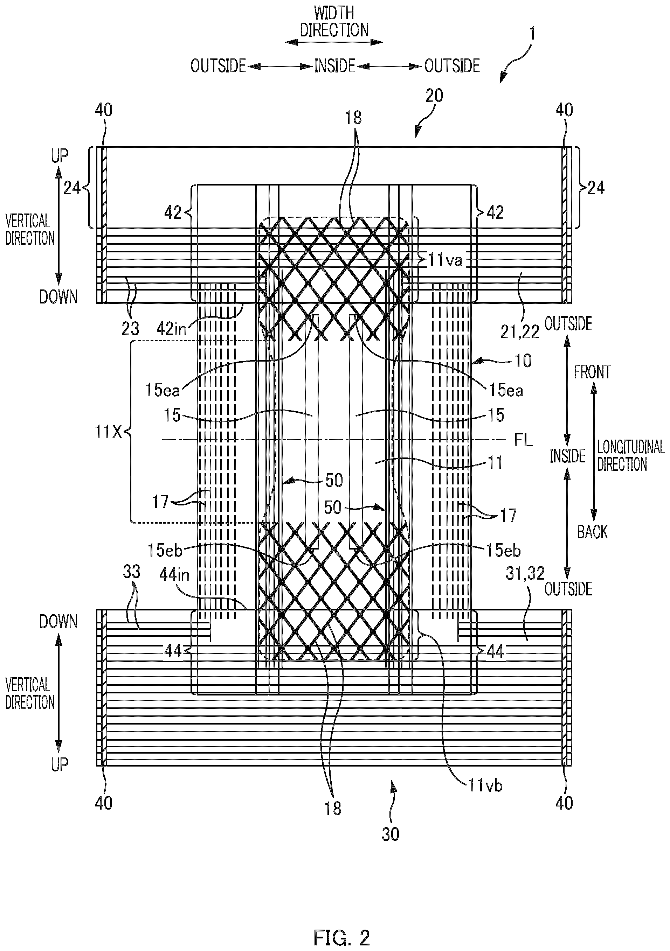

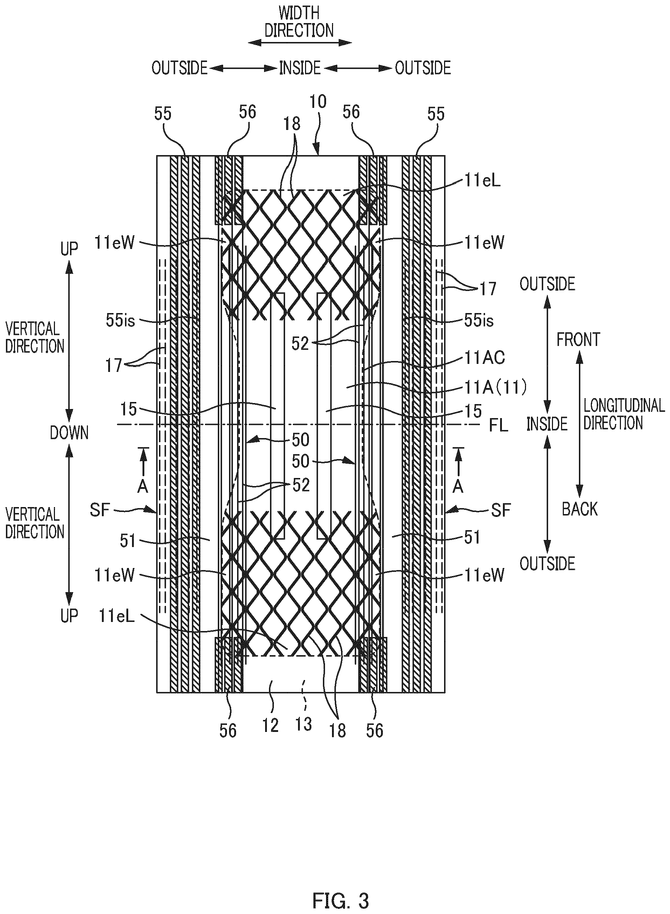

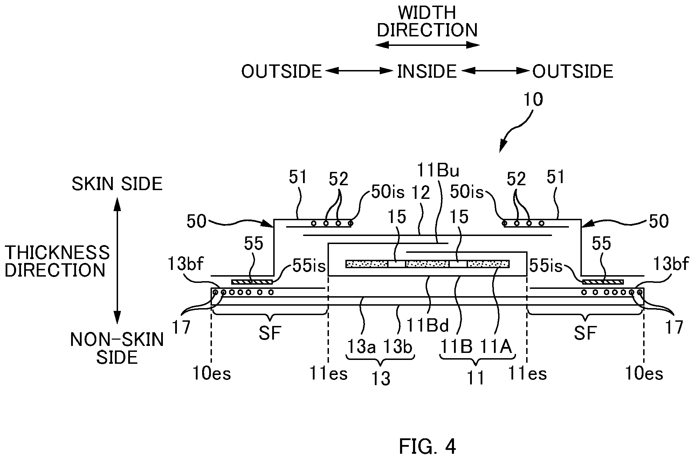

FIG. 1 is a schematic perspective view of a pull-on disposable diaper 1 (hereinafter referred to as "diaper") . FIG. 2 is a schematic plan view of the diaper 1 in an unfolded and stretched state. FIG. 3 is a plan view illustrating the configuration of an absorbent main body 10 and leak-proof wall portions 50. FIG. 4 is a schematic cross-sectional view taken along a line A-A of FIG. 3. The "unfolded state" of the pull-on disposable diaper is a state in which a waist joining portion 40, which will be described later, has been pulled apart into a pair of waist portions 20 and 30, and the diaper 1 has been opened in the longitudinal direction.

In the underpants-shaped state shown in FIG. 1, the diaper 1 has a vertical direction, a width direction, and a front-back direction that intersect each other, and a waist opening BH and a pair of leg openings LH have been formed. Also, in the unfolded state shown in FIG. 2, the diaper 1 has a longitudinal direction, a width direction, and a thickness direction that intersect each other. The longitudinal direction is a direction that extends along the vertical direction of the underpants-shaped state. The thickness direction is the direction in which members constituting the diaper 1 are overlaid on each other (see FIG. 4) . In the vertical direction, the waist opening BH side is the upper side, and the crotch side is the lower side. In the front-back direction, the stomach side of the wearer is the front side, and the back side of the wearer is the back side. In the thickness direction, the side that comes into contact with the wearer's body (skin) is the skin side, and the side that does not come into contact with the wearer's body (skin) is the non-skin side.

Also, the "stretched state" of the diaper 1 refers to a state in which, by stretching elastic members (e.g., leg elastic members 17 and waist elastic members 23 and 33) of the diaper 1, the whole diaper 1 (whole product) has been stretched to eliminate wrinkles, or more specifically, has been stretched such that the dimensions of constituent members of the diaper 1 (e.g., a later-described absorbent main body 10 and waist portions 20 and 30) match or are close to the dimensions of such members on their own.

Also, the diaper 1 has a pair of waist portions 20 and 30 and an absorbent main body 10. Out of the pair of waist portions 20 and 30, the one that is placed against the stomach side of the wearer is referred to as the front waist portion 20, and the one that is placed against the back side of the wearer is referred to as the back waist portion 30.

In the unfolded state shown in FIG. 2, the longitudinal one-side end portion of the absorbent main body 10 is arranged on the widthwise central portion of the front waist portion 20, and the diaper 1 has a first joining portion 42 in which the non-skin-side surface of the longitudinal one-side end portion and the front waist portion 20 are joined using an adhesive such as a hot-melt adhesive. Similarly, the longitudinal other-side end portion of the absorbent main body 10 is arranged on the widthwise central portion of the back waist portion 30, and the diaper 1 has a second joining portion 44 in which the non-skin-side surface of the longitudinal other-side end portion and the back waist portion 30 are joined using an adhesive such as a hot-melt adhesive . The absorbent main body 10 of the diaper 1 in the unfolded state is folded one time at a central position FL in the longitudinal direction thereof, and the two widthwise side portions of the front waist portion 20 and the two widthwise side portions of the back waist portion 30 are joined in a pair of waist joining portions 40, thus obtaining the diaper 1 in the underpants-shaped state shown in FIG. 1. Examples of the joining method used in the waist joining portions 40 include welding and joining with an adhesive.

As shown in FIG. 4, the absorbent main body 10 includes: an absorbent body 11; a liquid-permeable top sheet 12 arranged on the skin side of the absorbent body 11; and a back sheet 13 that is arranged on the non-skin side of the absorbent body 11 and forms the exterior of the absorbent main body 10. Also, a pair of leak-proof wall portions 50 are provided on respective sides of the absorbent body 11 in the width direction.

The absorbent body 11 has an absorbent core 11A that absorbs and holds an excreted fluid such as urine, and a liquid-permeable core-wrapping sheet 11B that covers the absorbent core 11A. The absorbent core 11A can be obtained by molding liquid absorbent fibers such as pulp containing a super absorbent polymer (SAP) into a predetermined shape, for example. On the other hand, the core-wrapping sheet 11B can be made of tissue paper, for example.

The absorbent core 11A of the present embodiment includes a narrow portion 11AC, which has a smaller length in the width direction (smaller width) than the front end portion and the back end portion in the longitudinal direction (the front upper end portion and the back upper end portion in the vertical direction), at a location between the front end portion and the back end portion, and thus has a substantially hourglass-like shape in a plan view as shown in FIG. 3. The narrow portion 11AC is the portion that is sandwiched between the wearer's legs when wearing the diaper 1, and the absorbent core 11A more easily fits to the wearer's crotch due to the decreased length in the width direction (decreased width) of this portion.

Also, in the present embodiment, the absorbent body 11 (absorbent core 11A) has a pair of low-basis-weight portions 15 in the widthwise central portion, each low-basis-weight portion 15 being a portion that extend in the longitudinal direction. Here, the basis weight is the mass per unit area, and the low-basis-weight portions 15 are portions where the basis weight is smaller than the surrounding area. The low-basis-weight portions 15 of the present embodiment also include the case where the basis weight is zero. Because the absorbent body 11 has the low-basis-weight portions 15, the absorbent body 11 can be induced to deform at the low-basis-weight portions 15 so as to fit to the wearer's crotch region. Also, when the wearer urinates, the urine can be quickly dispersed in the longitudinal direction along the low-basis-weight portions 15. Note that the number of and arrangement of the low-basis-weight portions 15 are not limited to the configuration of the low-basis-weight portions 15 of the present embodiment, and it is preferable to provide a pair of low-basis-weight portions 15 that extend along the longitudinal direction. For example, in the case where only one low-basis-weight portion 15 is provided, if the urination position shifts to the left or right, it is difficult to obtain the effect of dispersion by the low-basis-weight portion 15, but if two are provided, the effect of dispersion by the low-basis-weight portions 15 can be obtained even when the wearer sleeps on their side, for example. Also, if a pair of low-basis-weight portions 15 are provided with a gap therebetween in the width direction, the central portion in the width direction between the pair of low-basis-weight portions 15 can easily deform into a cup shape, and the absorbent body 11 can be easily induced to deform so as to conform to the wearer's body.

When the pair of low-basis-weight portions 15 are arranged in the absorbent body 11, the length (gap) between the low-basis-weight portions 15 in the width direction is preferably 25 mm or more, and more preferably 30 mm or more. According to this configuration, it is possible to ensure the absorption capacity in the vicinity of the urination orifice of the wearer. Also, the width of each of the low-basis-weight portions 15 is preferably 5 to 20 mm, and more preferably 8 to 12 mm. If the width is less than 5 mm, it is difficult to maintain the shape of the low-basis-weight portion 15, and the effect of dispersing the excreted fluid is also unlikely to be exhibited. On the other hand, if the width is more than 20 mm, the absorption capacity in the vicinity of the urinary orifice may decrease, and the movement of excreted fluid to adjacent portions of the absorbent body 11 may be hindered.

Also, as shown in FIG. 2, the absorbent body 11 has compressed portions 18, in which the absorbent core 11A is embossed, on both sides in the longitudinal direction. Details of the compressed portions 18 will be described later.

The top sheet 12 is a sheet-like member that covers the absorbent body 11 from the skin side, and is formed of, for example, a liquid-permeable nonwoven fabric sheet whose planar size is larger than that of the absorbent body 11.

The back sheet 13 is a sheet-like member that covers the absorbent body 11 from the non-skin side, and is a sheet member whose planar size is larger than that of the absorbent body 11. The back sheet 13 is a sheet member having a two-layer structure that includes: a back film 13a, which is a liquid-impermeable leak-proof sheet made of polyethylene, polypropylene, or the like; and an exterior sheet 13b that is made of nonwoven fabric or the like and is provided on the non-skin side of the back film 13a, for example.

The exterior sheet 13b has extension portions 13bf that extend a large amount outward from two widthwise ends 11es of the absorbent body 11 (see FIG. 3) . These extension portions 13bf are folded back inward in the width direction and to the skin side in the thickness direction, at two widthwise ends 10es of the absorbent main body 10 so as to be overlaid in the thickness direction. Thereby, side flaps SF are formed. In the side flaps SF, a plurality of leg elastic members 17, which are elastic strings or the like, are stretched in the longitudinal direction (vertical direction) and fixed at predetermined intervals in the width direction between portions of the exterior sheet 13bf that was folded in the thickness direction. Accordingly, the side flaps SF are given stretchability along the vertical direction (the longitudinal direction of the absorbent main body 10).

The leak-proof wall portions 50 are provided at locations that are on both widthwise sides of the absorbent body 11 and on the skin side in the thickness direction. When the diaper 1 is put on, the leak-proof wall portions 50 rise toward the wearer's skin from both widthwise sides of the absorbent body 11, thus suppressing the leakage of excrement to the outside of the absorbent main body 10.

The leak-proof wall portions 50 of the present embodiment each have a leak-proof-wall sheet 51 and a plurality of leak-proof-wall elastic members 52. The leak-proof-wall sheet 51 is a flexible rectangular sheet member, and is made of nonwoven fabric, for example. Also, the leak-proof-wall sheets 51 are joined along the vertical direction (the longitudinal direction of the absorbent main body 10) to the two widthwise end portions of the absorbent main body 10, and as shown in FIG. 4, are folded outward in the width direction at widthwise inner ends 50is. A plurality of leak-proof-wall elastic members 52, which are elastic strings or the like, are stretched in the vertical direction and fixed at predetermined intervals in the width direction between the portions of the leak-proof-wall sheets 51 that were folded at the inward ends 50is and are overlapped in the thickness direction.

Also, as shown by the hatched portions in FIG. 3, the leak-proof wall portions 50 are joined to the absorbent main body 10 by first joining regions 55 and second joining regions 56 that are formed by a joining material such as a hot-melt adhesive. The first joining regions 55 are band-shaped regions that extend along the vertical direction (the longitudinal direction of the absorbent main body 10) at outward end portions of the leak-proof wall portion 50 in the width direction, and are for joining the leak-proof wall portions 50 to the skin-side surfaces of the side flaps SF. The second joining regions 56 are provided in the two end portions of the absorbent main body 10 in the longitudinal direction (the upper end portions in the vertical direction), and are for joining the two longitudinal end portions of the leak-proof wall portions 50 to the skin-side surface of the top sheet 12.

Due to the leak-proof-wall elastic members 52, the leak-proof wall portions 50 are given stretchability along the vertical direction (longitudinal direction), and when the diaper 1 is put on, the widthwise inward end portions (inward ends 50is) of the leak-proof wall portion 50 rise toward the wearer's skin due to this stretchability. In other words, the first joining regions 55 function as base end portions that cannot rise in the thickness direction of the leak-proof wall portion 50, and there are provided rising portions (50is), which can rise from the base end portions to the skin side in the thickness direction (55) due to the leak-proof-wall elastic members 52 that can stretch and contract in the longitudinal direction.

The front waist portion 20 and the back waist portion 30 respectively have inner-layer sheets 21 and 31, outer-layer sheets 22 and 32 that are overlaid on the inner-layer sheets 21 and 31, and a plurality of waist elastic members 23 and 33.

The outer-layer sheets 22 and 32 are arranged on the non-skin side of the inner-layer sheets 21 and 31. The inner-layer sheets 21 and 31 and the outer-layer sheets 22 and 32 are arranged on the non-skin side of the absorbent main body 10. The inner-layer sheets 21 and 31 and the outer-layer sheets 22 and 32 on their own are non-stretchable sheets that substantially do not have stretchability in the width direction of the diaper 1. The inner-layer sheets 21 and 31 and the outer-layer sheets 22 and 32 can be constituted by SMS nonwoven fabric (spunbond-meltblown-spunbond nonwoven fabric), spunbond nonwoven fabric, air-through nonwoven fabric, a plastic sheet, a perforated plastic sheet, a laminated sheet including any of the foregoing, or the like.

The waist elastic members 23 and 33 are arranged side by side in the vertical direction between the inner-layer sheets 21 and 31 and the outer-layer sheets 22 and 32, and are fixed in a state of being stretched in the width direction. Accordingly, the front waist portion 20 and the back waist portion 30 stretch and contract in the width direction to fit the wearer's waist. Examples of the waist elastic members 23 and 33 include thread-like elastic members made of rubber, spandex, or the like.

In the back waist portion 30 of the present embodiment, the waist elastic members 33 are arranged in a region extending from the upper end portion to the lower end portion. On the other hand, the waist elastic members 23 are not arranged in the upper end portion of the front waist portion 20, but rather a stretchable sheet 24 that stretches and contracts in the width direction is provided. In this way, by arranging the stretchable sheet 24 instead of waist elastic members 23 in the upper end portion of the front waist portion 20, the upper end portion of the front waist portion 20 is more likely to come into flat contact with the wearer's body (skin).

Next, the compressed portions 18 will be described in detail. In the absorbent body 11, the absorbent core 11A has a plurality of linear compressed portions 18. In these "compressed portions", the absorbent core 11A has been compressed in the thickness direction by embossing. Specifically, the linear compressed portions 18 each have two ends, namely one end and another end, are arranged so as to diagonally intersect each other in the longitudinal direction and the width direction, and have a predetermined length in the longitudinal direction and the width direction. There are also a plurality of substantially diamond-shaped inner regions that are surrounded on all sides by the compressed portions 18. Due to such compression, the fiber density of the absorbent core 11A is higher than that of the uncompressed portions, and the capillary effect facilitates the absorption of excrement in the compressed portions 18. Note that the compressed portions 18 of the present embodiment are shown by bold solid lines in FIGS. 2 and 3, but this is for convenience in the description, and because the absorbent core 11A is actually embossed as described above, the compressed portions are not clearly visible from the skin-side surface of the diaper 1.

Also, although the compressed portions 18 of the present embodiment are formed linearly, the linear compressed portions 18 may each be formed by a set of dot-like (circular) compressed portions. Alternatively, dot-like compressed portions or linear compressed portions may be formed discretely in a predetermined region. Also, as one example of a formation patterns for the compressed portions 18, the compressed portions 18 may be arranged so as to look like a lattice pattern (so-called quilting pattern) overall as shown in FIG. 2, but other patterns may be used. Also, the lines that form the lattice pattern may be straight lines as shown in FIG. 2, or may be curved lines.

In the compressed portions 18 of the present embodiment, the absorbent core 11A is in a state of being compressed in the thickness direction, but it is sufficient that at least the absorbent core 11A is compressed in the thickness direction, and alternatively, the absorbent core 11A, the core-wrapping sheet 11B, and the top sheet 12 may be compressed in the thickness direction, for example. In other words, the absorbent core 11A, the core-wrapping sheet 11B (absorbent body 11), and the top sheet 12 may have the compressed portions 18.

In the diaper 1 of the present embodiment, the absorbent body 11 is provided with the low-basis-weight portions 15 that extend along the longitudinal direction, and when the wearer excretes a fluid, the excreted fluid can be guided and dispersed forward and backward in the longitudinal direction of the absorbent body 11 along the low-basis-weight portions 15. The compressed portions 18 of the absorbent body 11 are portions where the fiber density of the absorbent core 11A is higher than that of the uncompressed portions, but in the low-basis-weight portions 15, the basis weight is smaller than the surrounding area or is zero, and therefore the portions of the compressed portions 18 that are not overlapped with the low-basis-weight portions 15 in a view in the thickness direction are portions that have a high stiffness. Specifically, in the diaper 1, the portions of the compressed portions 18 that are not overlapped with the low-basis-weight portions 15 in a view in the thickness direction are high-stiffness portions that have a higher stiffness than that at the central position FL (FIG. 2) at which the diaper 1 is folded. Here, "at the central position FL" is an area sandwiched by the pair of low basis-weight regions 15 at the central position FL (also referred to as the center line FL) at which the diaper 1 is folded, and is an area that extends 1 cm both forward and backward in the longitudinal direction from the center line.

The stiffness values of the compressed portions 18 and the central position FL region of the diaper 1 can be obtained by, for example, measuring Gurley stiffness values and dividing such values by the length of the sample piece. The Gurley stiffness can be measured in accordance with JIS-L1096, for example, using a No. 311 Gurley stiffness tester manufactured by Yasuda Seiki Seisakusho, Ltd. Note that in the present embodiment, it is sufficient to be able to check the magnitude relationship of the stiffness between the compressed portions 18 and the central position FL, and therefore it is not necessary to obtain an exact value, and a certain level of error in the measured values is permissible.

The low-basis-weight portions 15, which are arranged at least in the longitudinal central portion that corresponds to the crotch region of the wearer, guide and disperse the absorbed excreted fluid in the longitudinal direction while also serving as folding points for deformation of the absorbent body 11, thus allowing the absorbent body 11 to fit the wearer's body (crotch region) . On the other hand, if the low-basis-weight portions 15 extend up to or near the front waist portion 20 or the back waist portion 30, deformation due to folding may extend up to the first joining portion 42 (the second joining portion 44) that joins the absorbent main body 10 and the front waist portion 20 (the back waist portion 30), or more specifically up to the longitudinal end portions of the absorbent body 11, and leakage can possibly occur.

In view of this, as shown in FIG. 2, the diaper 1 of the present embodiment has a high-stiffness portion, which has a higher stiffness than at the central position FL, in at least one of (in the present embodiment, in both of) a longitudinal region extending from an inward end 42in of the first joining portion 42 to outward ends 15ea of the low-basis-weight portions 15 and a longitudinal region extending from an inward end 44in of the second joining portion 44 to outward ends 15eb of the low-basis-weight portions 15. Accordingly, after urination, urine is rapidly dispersed throughout the absorbent body 11 along the low-basis-weight portions 15, and the high-stiffness portion can suppress deformation of the absorbent body 11 outward of the low-basis-weight portions 15 in the longitudinal direction, because the high-stiffness portion is located outside in the longitudinal direction with respect to the outward ends 15ea (15eb) of the low-basis-weight portions 15. Accordingly, by preventing deformation up to the longitudinal end portions of the absorbent main body 10, it is possible to suppress the occurrence of leakage.

Also, it is preferable that the low-basis-weight portions 15 and the high-stiffness portions are provided on extension lines along the longitudinal direction. Specifically, it is preferable to have a portion where the low-basis-weight portions 15 and the high-stiffness portions are overlapped in the width direction. Even if the high-stiffness portions are arranged at positions that are not on extension lines of the low-basis-weight portions 15 in the longitudinal direction, it is possible to suppress deformation of the absorbent body 11 if the high-stiffness portions are located outward of the low-basis-weight portions 15 in the longitudinal direction. However, if the high-stiffness portions are provided on extension lines of the low-basis-weight portions 15 in the longitudinal direction, it is possible to more reliably induce deformation of the absorbent body 11 at the low-basis-weight portions 15.

Also, as shown in FIG. 2, the diaper 1 of the present embodiment has a non-compressed region 11X, which does not include the compressed portions 18, in a predetermined range that includes the central position FL at which the diaper 1 is folded in the longitudinal direction. The "predetermined range that includes the central position FL" is a region that extends a predetermined width forward and backward in the longitudinal direction from the central position FL, and is not limited to the range shown in FIG. 2. The non-compressed region 11X is a portion that comes into contact with the wearer's crotch when the diaper 1 is put on, and due to not providing the compressed portions 18 in the crotch portion, it is possible to further improve the ability to immediately absorb excreted urine and to swiftly disperse the urine in the longitudinal direction with use of the low-basis-weight portions 15. Also, by providing the non-compressed region 11X at the crotch, there is no portion having high stiffness (so-called high-stiffness portion), and therefore the diaper can more easily fit to the crotch region when the wearer closes their legs, for example.

Also, as described above, the portions of the compressed portions 18 that are not overlapped with the low-basis-weight portions 15 in a view in the thickness direction are the high-stiffness portions, that is to say, in the diaper 1 of the present embodiment, the high-stiffness portion is provided in at least part of a portion 11va of the absorbent body 11 that is overlapped with the first joining portion 42 in a view in the thickness direction or a portion 11vb of the absorbent body 11 that is overlapped with the second joining portion 44 in a view in the thickness direction. In the present embodiment, in the first joining portion 42 and the second joining portion 44, adhesive (not shown) for joining the longitudinal end portions of the absorbent main body 10 to the waist portions (front waist portion 20 and back waist portion 30) is provided extending along the longitudinal direction, at intermittent locations in the width direction. By providing the adhesive intermittently, it is possible to suppress the case where the first joining portion 42 and the second joining portion 44 become too hard. The portions where the adhesive of the first joining portion 42 or the second joining portion 44 are overlapped with the compressed portions 18 in a view in the thickness direction has an even higher stiffness, thus making it possible to more reliably suppress deformation of the low-basis-weight portions 15. In other words, deformation at the low-basis-weight portions 15 is suppressed by the high-stiffness portions located in the region extending from the inward end 42in (44in) of the first joining portion 42 to the outward ends 15ea (15eb) of the low-basis-weight portions 15, and furthermore, deformation can be more reliably suppressed by the portion where the high-stiffness portion is overlapped with the adhesive of the first joining portion 42 or the second joining portion 44 in a view in the thickness direction. Therefore, deformation can be suppressed neatly without forming unnatural folds, wrinkles, or the like due to rapid suppression. Also, by providing the adhesive intermittently in the width direction, it is possible to prevent the entirety of the first joining portion 42 and the second joining portion 44 from becoming excessively hard even if they are overlapped with the compressed portions 18, thus making it unlikely for the wearer to feel uncomfortable. Note that the arrangement of the adhesive in the first joining portion 42 and the second joining portion 44 is not limited to the arrangement of the present embodiment, and the adhesive can be applied in any pattern.

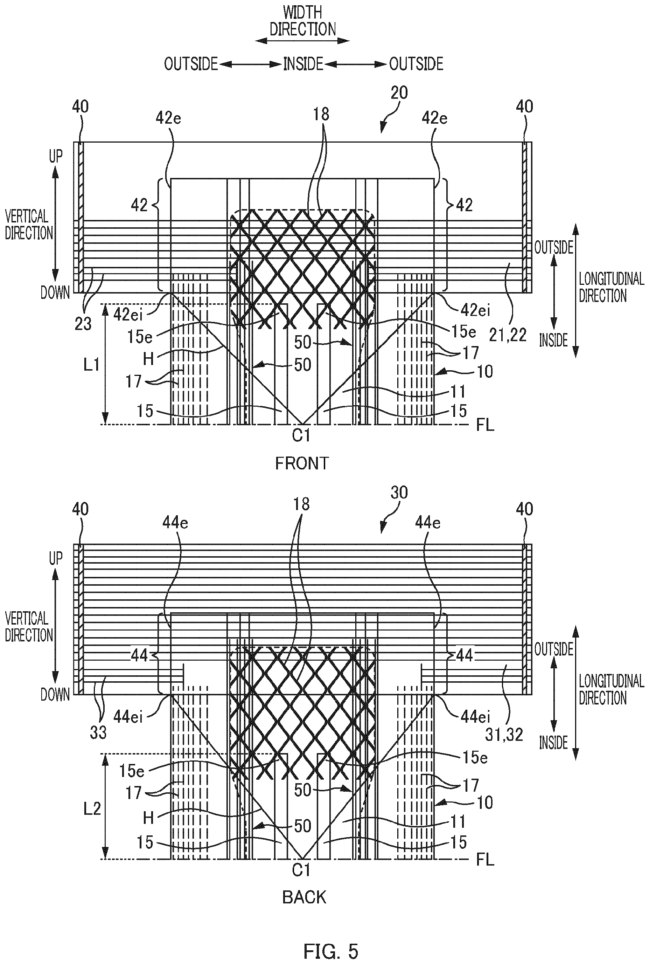

FIG. 5 is a schematic plan view of virtual lines H on the front side and the back side of the diaper 1. In the front-side view in FIG. 5, virtual lines H are drawn so as to connect the positions of longitudinal inward ends 42ei of two widthwise ends 42e of the first joining portion 42 to a widthwise center point C1 located at the longitudinal central position FL of the diaper 1. Also, in the back-side view in FIG. 5, virtual lines H are drawn so as to connect the positions of longitudinal inward ends 44ei of two widthwise ends 44e of the second joining portion 44 to the widthwise center point C1 located at the longitudinal central position FL of the diaper 1. When the user is wearing the diaper 1, fold lines are likely to form in the absorbent main body 10 along at least a part of the virtual lines H after urination. In the present embodiment, the low-basis-weight portions 15 longitudinally extend to positions outward in the longitudinal direction beyond the virtual lines H. Also, the compressed portions 18 are located in portions that are overlapped with longitudinal end portions 15e of the low-basis-weight portions 15 in a view in the thickness direction (i.e., the direction passing through the paper surface). If the embossed compressed portions 18 and the low-basis-weight portions 15 overlap in this way, the compressed portions 18 are normally likely to become separated at the locations overlapped with the low-basis-weight portions 15. But here, even if the compressed portions 18 are linear due to the separated portions being connected by extension lines, it can be deemed that embossing has been performed.

If the low-basis-weight portions 15 located at positions below the virtual lines H (inward thereof in the longitudinal direction), there would be a risk that the dispersion of excreted fluid by the low-basis-weight portions 15 would be stopped by creases formed along the virtual lines H. However, if the low-basis-weight portions 15 extend to the same position as the virtual lines H or outward beyond the virtual lines H in the longitudinal direction (upward in the vertical direction), the dispersion of excreted fluid is less likely to be stopped. Also, because the compressed portions 18 are located in portions overlapped with the end portions 15e of the low-basis-weight portions 15 in the thickness direction, the liquid lifting capability is improved at the end portions 15e of the low-basis-weight portions 15, and the liquid transmitted by the low-basis-weight portions 15 can be further dispersed in the compressed portions 18.

Also, as shown in FIG. 5, in the state where the absorbent main body 10 has been folded at the central position FL, the side on which the front waist portion 20 and the back waist portion 30 are arranged is the upper side in the vertical direction, and the central position FL side is the lower side. In this vertical direction, the length L1 of the low-basis-weight portions 15 on the front side (see the stomach-side view in FIG. 5) is longer than the length L2 of the low-basis-weight portions 15 on the back side (the back-side view in FIG. 5) . On the front side near the urination orifice, setting a longer length for the low-basis-weight portions 15 makes excreted urine move vertically along the low-basis-weight portions 15, making it possible to improve the dispersion of excreted fluid. On the other hand, on the back side, if the low-basis-weight portions 15 are too long, there is a risk that the low-basis-weight portions 15 promotes deformation of the absorbent main body 10, and that it is not possible to secure a sufficient stool storage space. Therefore it is preferable that the length on the back side is set so as to not be too long.

Also, in the present embodiment, as shown in the back-side view in FIG. 5, portions of the lower end portions of compressed portions 18 are provided so as to cross the virtual lines H in a plan view. As described above, in a plan view, compressed portions 18 are provided so as to cross at least a part of the virtual lines H, and therefore the folding of the absorbent body 11 along the virtual lines H, which tends to occur after urination, is suppressed, and it is possible to prevent the dispersion of excreted fluid from being stopped due to such folding.

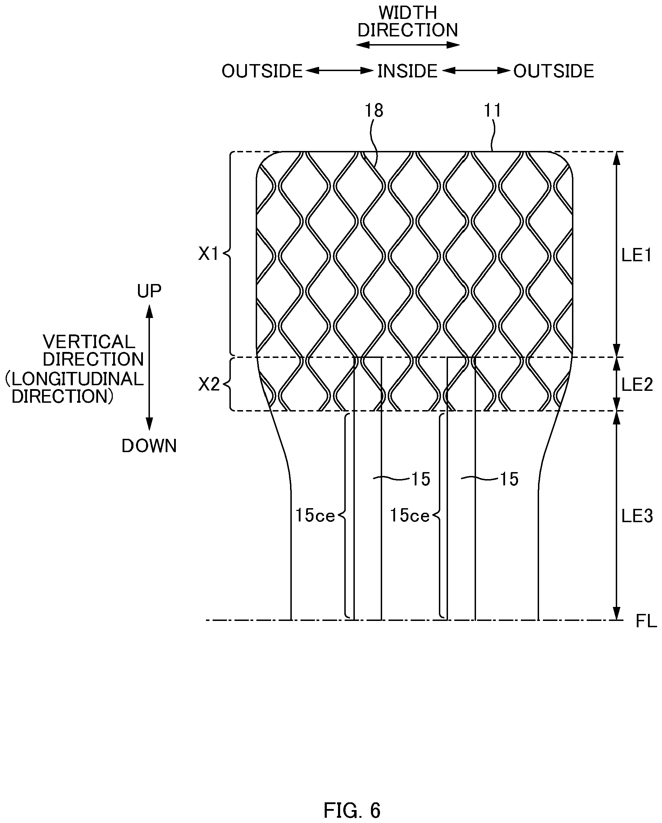

FIG. 6 is a plan view showing only the front side of the absorbent body 11 when the diaper 1 has been folded at the central position FL in the longitudinal direction. In order to facilitate the description of the compressed portions 18 and the pair of low-basis-weight portions 15, only the absorbent body 11 is shown in this figure. As shown in FIG. 6, with respect to the longitudinal direction, there is an overlap region X2 that includes compressed portions 18 being overlapped with the low-basis-weight portions 15, and a non-overlap region X1 that includes compressed portions 18 and that is located outward (vertically upward) of the overlap region X2. The length LE2 of the overlap region X2 is shorter than the length LE1 of the non-overlap region X1 (LE1 > LE2). The overlap region X2 is a region where excreted fluid that has moved along the low-basis-weight portions 15 is passed to the non-overlap region X1. The non-overlap region X1 is a region where the low-basis-weight portions 15 do not exist, and because the compressed portions 18 in the non-overlap region X1 are high-stiffness portions, the non-overlap region X1 is a region that suppresses deformation of the absorbent body 11 while also allowing excreted fluid to be dispersed over an even wider area due to the excreted-fluid lifting effect of the compressed portions 18. If the overlap region X2 does not exist, it may be difficult for excreted fluid to be properly passed to the non-overlap region X1, and therefore it is necessary to provide the overlap region X2 with at least a minimum required size. Also, if the overlap region X2 is longer than the non-overlap region X1 (LE1 < LE2), the low-basis-weight portions 15 that serve as folding points of the absorbent body 11 will be longer, deformation of the absorbent body 11 will be promoted more than necessary, and there will be an increased possibility of allowing leakage. Not only in the case where the length LE2 of the overlap region X2 is set shorter than the length LE1 of the non-overlap region X1 as in the present embodiment, that is to say, if the overlap region X2 is provided such that the longitudinal length of the overlap region X2 is less than or equal to the longitudinal length of the non-overlap region X1, the smaller overlap region X2 increases the possibility that liquid is smoothly moved by the low-basis-weight portions 15 and dispersed by the non-overlap region X1, compared with the case where the overlap region X2 is provided such that the longitudinal length LE2 of the overlap region X2 is longer than the longitudinal length LE1 of the non-overlap region X1.

Also, as shown in FIG. 6, letting low-basis-weight-portion central regions 15ce be regions of the low-basis-weight portions 15 that extend in the vertical direction from the lower end of the overlap region X2 to the central position FL, the length LE3 of the low-basis-weight-portion central regions 15ce is longer than the length LE2 of the overlap region X2. The low-basis-weight-portion central regions 15ce are regions that are close to the wearer's urination orifice, and if the overlap region X2 is longer than the low-basis-weight-portion central regions 15ce, the immediate absorption performance near the urination orifice deteriorates, and leakage may occur. For this reason, by setting the length LE3 of the low-basis-weight-portion central regions 15ce longer than the length LE2 of the overlap region X2, excreted urine is rapidly moved in the longitudinal direction by the low-basis-weight portions 15, and excreted fluid is passed in the overlap region X2, and thus excreted fluid can be easily dispersed over a wide area.

Also, in the absorbent body 11 shown in FIG. 6, it is preferable that the length LE1 of the non-overlap region X1 is longer than the length LE3 of the low-basis-weight-portion central regions 15ce. If the low-basis-weight-portion central regions 15ce extend too far to the front side, the region that can absorb excreted fluid such as urine that has been moved and dispersed becomes insufficiently large, and leakage may occur. Also, if the low-basis-weight-portion central regions 15ce extend too far to the back side, deformation of the absorbent main body 10 (absorbent body 11) is promoted, and there is a risk that the stool storage space cannot be sufficiently secured and leakage will occur. Accordingly, by setting the non-overlap region X1 longer while also providing the low-basis-weight-portion central regions 15ce over an appropriate range, after a liquid has been rapidly dispersed by the low-basis-weight portions 15, it is possible to promote the dispersion of the liquid over a wider area by the liquid lifting effect of the non-overlap region X1 that is provided over a wider area.

Also, in the present embodiment, the non-overlap region X1 extends up to the longitudinal end portion (end portion 11eL in FIG. 3) or up to the widthwise end portion (end portion 11eW in FIG. 3) of the absorbent body 11. Accordingly, excreted fluid that has been moved in the longitudinal direction by the low-basis-weight portions 15 can be widely dispersed up to the longitudinal end portion 11eL or the widthwise end portion 11eW of the absorbent body 11 by the capillary effect of the compressed portions 18 in the non-overlap region X1, and more efficient absorption can be realized.

Also, in the present embodiment, the high-stiffness portions having the compressed portions 18 may be linear as described above or be formed by dots. More specifically, the high-stiffness portions have a portion in which a high-density portion is formed in a linear manner, the high-density portion being a portion whose density is higher than the surrounding area, but the high-stiffness portion is not limited to this. The high-stiffness portion may have a portion in which a plurality of high-density portions each having a dot-like shape are formed, the high-density portion being a portion whose density is higher than the surrounding area. With the portion in which the high-density portion is formed in a linear manner, it is possible to disperse excreted fluid to a greater degree along the linear high-density portion. Also, it is possible to adjust the stiffness to any strength by using a linear pattern. With the portion in which the plurality of high-density portions each having a dot-like shape are formed, each dot-shaped high-density portion serves as a starting point for absorbing a liquid, and the excreted fluid can be dispersed by spreading out from each dot.

Also, returning to FIG. 4, the absorbent body 11 has the absorbent core 11A and the core-wrapping sheet 11B. In the low-basis-weight portions 15 of the present embodiment, a core-wrapping sheet 11Bu located on the skin side in the thickness direction and a core-wrapping sheet 11Bd located on the non-skin side are joined using an adhesive. If the core-wrapping sheets 11Bu and 11Bd on the skin side and the non-skin side are not joined to each other in the low-basis-weight portions 15, the absorbent body 11 swells when urine is absorbed, and the interior constituent materials of the absorbent core 11A may move, and there is a risk that the low-basis-weight portions 15 will eliminate due to such factors. By joining the core-wrapping sheets 11Bu and 11Bd on the skin side and the non-skin side to each other, it is possible to prevent the low-basis-weight portions 15 from being eliminated, and the effects of movement and dispersion of excreted fluid by the low-basis-weight portions 15 can be realized continuously over time.

### Other embodiments

Although the embodiment of the present disclosure has been described hereinabove, the above embodiment of the present disclosure is simply to facilitate understanding of the present disclosure and are not in any way to be construed as limiting the present disclosure. The present disclosure may variously be changed or altered without departing from its gist and encompass equivalents thereof.

In the above-described embodiment, as shown in FIG. 3, the compressed portions 18 are provided up to the end portions 11eW and 11eW of the absorbent body 11 in the width direction, but the present invention is not limited to this. A configuration is possible in which, in the diaper 1 that has the leak-proof wall portions 50, the compressed portions 18 are provided, in the width direction, in the entirety of a region that extends from an inward end 55is of one base end portion (55) of the leak-proof wall portions 50 to the inward end 55is of the other base end portion (55). In order to block excreted urine, it is preferable that the region inward of the leak-proof wall portions 50 in the width direction does not deform. If the compressed portions 18 are provided between the base end portions 55, such deformation is suppressed, and the leak prevention function can be further improved.

In the above-described embodiment, the high-stiffness portions have the embossed compressed portions 18, but the present invention is not limited to this. The high-stiffness portions may be formed by increasing the basis weight of the absorbent core 11A, or may be formed by affixing another material.

Also, in the above embodiment, a diaper in which the front waist portion 20 and the back waist portion 30 are separate members is illustrated, but a configuration is possible in which a crotch portion is provided between the front waist portion 20 and the back waist portion 30 such that the front waist portion 20 and the back waist portion 30 are a single continuous member.

Also, in the above embodiment, the absorbent core 11A has the narrow portion 11AC between the front end portion and the back end portion in the longitudinal direction, but the present invention is not limited to this. For example, the absorbent core 11A (absorbent body 11) may be rectangular. Giving the absorbent core 11A a rectangular shape makes it possible to maintain the volume of excreted fluid that can be absorbed.

### Reference Signs

1 pull-on disposable diaper (underpants-shaped absorbent article),
10 absorbent main body, 10es widthwise ends,
11 absorbent body, 11A absorbent core, 11AC narrow portion,
11B core-wrapping sheet, 11Bd core-wrapping sheet,
11Bu core-wrapping sheet, 11eL end portion, 11es end,
11eW end portion, 11va overlap portion, 11vb overlap portion,
11X non-compressed region,
12 top sheet, 13 back sheet,
13a back film, 13b exterior sheet,
13bf extension portion, 15 low-basis-weight portion,
15ce basis-weight-portion central region, 15e end portion,
15ea longitudinal outward end, 15eb longitudinal outward end,
17 leg elastic member, 18 compressed portion,
20 stomach-side waist portion, 21 inner-layer sheet, 22 outer-layer sheet,
23 waist elastic member, 24 stretchable sheet,
30 back waist portion, 31 inner-layer sheet, 32 outer-layer sheet,
33 waist elastic member, 40 waist joining portion,
42 first joining portion, 42e widthwise ends, 42ei inward end,
42in inward end, 44 second joining portion, 44e widthwise ends,
44ei inward end, 44in inward end,
50 leak-proof wall portion, 50is rising portion (inward end)
51 leak-proof-wall sheet, 52 leak-proof-wall elastic member,
55 first joining region, 55 base end portion (first joining region),
55is inward end, 56 second joining region

## Claims

1. An underpants-shaped absorbent article having a longitudinal direction, a width direction, and a thickness direction that intersect each other in an unfolded state,
the underpants-shaped absorbent article comprising:
a front waist portion (20);
a back waist portion (30);
an absorbent main body (10) that includes an absorbent body (11),
a first joining portion (42) in which a non-skin-side surface of an end portion of the absorbent main body (10) on one side in the longitudinal direction is joined to the front waist portion (20) using an adhesive; and
a second joining portion (44) in which a non-skin-side surface of an end portion of the absorbent main body (10) on another side in the longitudinal direction is joined to the back waist portion (30) using an adhesive,
the absorbent main body (10) being folded one time at a central position (FL) in the longitudinal direction,
widthwise end portions of the front waist portion (20) and widthwise end portions of the back waist portion (30) being joined in a pair of waist joining portions,
the absorbent body (11) including a low-basis-weight portion (15) in at least a central portion in the longitudinal direction,
the low-basis-weight portion (15) extending along the longitudinal direction,
the low-basis-weight portion (15) having a smaller basis weight than a surrounding area,
a high-stiffness portion being provided in at least one of
a longitudinal region extending from an inward end (42in) of the first joining portion (42) to an outward end (15ea) of the low-basis-weight portion (15) and
a longitudinal region extending from an inward end (44in) of the second joining portion (44) to another outward end (15eb) of the low-basis-weight portion (15),
the high-stiffness portion being a portion that has a higher stiffness than the central position (FL).

2. A underpants-shaped absorbent article according to claim 1, wherein
the high-stiffness portion includes a compressed portion (18), and
in a case where a virtual line (H) is drawn such that a position of a longitudinal inward end (42ei, 44ei) of each widthwise end (42e, 44e) of either of the first joining portion (42) and the second joining portion (44) is connected to a widthwise center point (C1) located at the central position (FL) in the longitudinal direction,
the low-basis-weight portion (15) extends in the longitudinal direction, to a position identical to the virtual line (H) or outward in the longitudinal direction beyond the virtual line (H), and
the compressed portion (18) is provided in a portion that is overlapped with a longitudinal end portion (15e) of the low-basis-weight portion (15) in a view in the thickness direction.

3. A underpants-shaped absorbent article according to claim 1 or 2, wherein
the high-stiffness portion includes a compressed portion (18), and
a non-compressed region (11X) is provided in a predetermined range that includes the central position (FL) in the longitudinal direction,
the non-compressed region (11X) being a region that does not include the compressed portion (18).

4. A underpants-shaped absorbent article according to any one of claims 1 to 3, wherein
the high-stiffness portion is provided in at least part of a portion of the absorbent body (11) that is overlapped with either of the first joining portion (42) and the second joining portion (44) in a view in the thickness direction.

5. A underpants-shaped absorbent article according to any one of claims 1 to 3, wherein
the adhesive in the first joining portion (42) and the second joining portion (44) is provided extending along the longitudinal direction, at intermittent locations in the width direction, and
the high-stiffness portion is provided in at least part of a portion of the absorbent body (11) that is overlapped with either of the first joining portion (42) and the second joining portion (44) in a view in the thickness direction.

6. A underpants-shaped absorbent article according to any one of claims 1 to 5, wherein
the high-stiffness portion includes a compressed portion (18), and
an overlap region (X2) and a non-overlap region (X1) are provided with respect to the longitudinal direction,
the overlap region (X2) being a region that includes the compressed portion (18) being overlapped with the low-basis-weight portion (15),
the non-overlap region (X1) being a region that includes the compressed portion (18) and that is located outward of the overlap region (X2), and
a longitudinal length (LE2) of the overlap region (X2) is less than or equal to a longitudinal length (LE1) of the non-overlap region (X1).

7. A underpants-shaped absorbent article according to any one of claims 1 to 6, wherein
the high-stiffness portion includes a compressed portion (18),
the underpants-shaped absorbent article has a vertical direction,
in the vertical direction, in a state where the absorbent main body (10) has been folded at the central position (FL),
a side on which the front waist portion (20) and the back waist portion (30) are arranged is an upper side, and
a side corresponding to the central position (FL) is a lower side,
an overlap region (X2) is provided with respect to the vertical direction,
the overlap region (X2) being a region that includes the compressed portion (18) being overlapped with the low-basis-weight portion (15), and
letting a low-basis-weight-portion central region (15ce) be a region of the low-basis-weight portion (15) that extends in the vertical direction from a lower end of the overlap region (X2) to the central position (FL),
a vertical length (LE3) of the low-basis-weight-portion central region (15ce) is longer than a vertical length (LE2) of the overlap region (X2).

8. A underpants-shaped absorbent article according to any one of claims 1 to 7, wherein
the high-stiffness portion includes a compressed portion (18),
the underpants-shaped absorbent article has a vertical direction,
in the vertical direction, in a state where the absorbent main body (10) has been folded at the central position (FL),
a side on which the front waist portion (20) and the back waist portion (30) are arranged is an upper side, and
a side corresponding to the central position (FL) is a lower side,
an overlap region (X2) and a non-overlap region (X1) are provided with respect to the vertical direction,
the overlap region (X2) being a region that includes the compressed portion (18) being overlapped with the low-basis-weight portion (15),
the non-overlap region (X1) being a region that includes the compressed portion (18) and that is located upward of the overlap region (X2), and
letting a low-basis-weight-portion central region (15ce) be a region of the low-basis-weight portion (15) that extends in the vertical direction from a lower end of the overlap region (X2) to the central position (FL),
a vertical length (LE1) of the non-overlap region (X1) is longer than a vertical length (LE3) of the low-basis-weight-portion central region (15ce).

9. A underpants-shaped absorbent article according to any one of claims 1 to 8, wherein
the high-stiffness portion has either of
a portion in which a high-density portion is formed in a linear manner, and
a portion in which the high-density portion is dot-shaped and a plurality of the high-density portions are formed,
the high-density portion being a portion whose density is higher than a surrounding area.

10. A underpants-shaped absorbent article according to any one of claims 1 to 9, wherein
the underpants-shaped absorbent article has a vertical direction,
in the vertical direction, in a state where the absorbent main body (10) has been folded at the central position (FL),
a side on which the front waist portion (20) and the back waist portion (30) are arranged is an upper side, and
a side corresponding to the central position (FL) is a lower side, and
letting a side on which the front waist portion (20) is located be a front side, and
letting a side on which the back waist portion (30) is located be a back side,
a vertical length of the low-basis-weight portion (15) on the front side is longer than a vertical length of the low-basis-weight portion (15) on the back side.

11. A underpants-shaped absorbent article according to any one of claims 1 to 10, wherein
the high-stiffness portion includes a compressed portion (18),
an overlap region (X2) and a non-overlap region (X1) are provided,
the overlap region (X2) being a region that includes the compressed portion (18) being overlapped with the low-basis-weight portion (15) in the longitudinal direction,
the non-overlap region (X1) being a region that includes the compressed portion (18) and that is located outward of the overlap region (X2) in the longitudinal direction, and
the non-overlap region (X1) extends up to either of a longitudinal end portion (11eL) and a widthwise end portion (11eW) of the absorbent body (11).

12. A underpants-shaped absorbent article according to any one of claims 1 to 10, wherein
the underpants-shaped absorbent article further comprises a pair of leak-proof wall portions (50) that are respectively provided on widthwise sides of the absorbent main body (10),
the pair of leak-proof wall portions (50) each include
a base end portion (55) that cannot rise in the thickness direction, and
a rising portion (50is) that can rise from the base end portion (55) to a skin side in the thickness direction due to an elastic member (52) that can stretch and contract in the longitudinal direction,
the high-stiffness portion includes a compressed portion (18), and
in the width direction, the compressed portion (18) is provided in an entirety of a region that extends from an inward end (55is) of one of the base end portions (55) to an inward end (55is) of another one of the base end portions (55).

13. A underpants-shaped absorbent article according to claim 2, wherein
the compressed portion (18) is provided so as to cross at least a portion of the virtual line (H) in a plan view.

14. A underpants-shaped absorbent article according to any one of claims 1 to 13, wherein
the absorbent body (11) includes an absorbent core (11A) and a liquid-permeable core-wrapping sheet (11B),
the core-wrapping sheet (11B) covering the absorbent core (11A) from a skin side and a non-skin side in the thickness direction, and
in the low-basis-weight portion (15),
a portion of the core-wrapping sheet (11B) located on the skin side is joined to a portion of the core-wrapping sheet (11B) located on the non-skin side.

## Patentansprüche

1. Unterhosen-förmiger absorbierender Artikel, der eine Längsrichtung, eine Breitenrichtung und eine Dickenrichtung aufweist, die in einem aufgefalteten Zustand einander schneiden,
wobei der Unterhosen-förmige absorbierende Artikel Folgendes umfasst:
einen vorderen Taillenteil (20);
einen hinteren Taillenteil (30);
einen absorbierenden Hauptkörper (10), der einen Saugkörper (11) einschließt,
einen ersten Verbindungsteil (42), in dem eine Nicht-Hautseitenoberfläche eines Endteils des absorbierenden Hauptkörpers (10) auf einer Seite in der Längsrichtung mit dem vorderen Taillenteil (20) unter Verwendung eines Haftmittels verbunden ist; und
einen zweiten Verbindungsteil (44), in dem eine Nicht-Hautseitenoberfläche eines Endteils des absorbierenden Hauptkörpers (10) auf einer anderen Seite in der Längsrichtung mit dem hinteren Taillenteil (30) unter Verwendung eines Haftmittels verbunden ist,
wobei der absorbierende Hauptkörper (10) an einer mittleren Position (FL) in der Längsrichtung einmal gefaltet ist,
Endteile in der Breite des vorderen Taillenteils (20) und Endteile in der Breite des hinteren Taillenteils (30) in einem Paar von Taillenverbindungsteilen verbunden sind,
der Saugkörper (11) einen Teil mit geringem Flächengewicht (15) in mindestens einem mittleren Teil in der Längsrichtung einschließt,
wobei sich der Teil mit geringem Flächengewicht (15) entlang der Längsrichtung erstreckt,
der Teil mit geringem Flächengewicht (15) ein kleineres Flächengewicht aufweist als eine umgebende Fläche,
wobei ein Teil mit hoher Steifigkeit in mindestens einem von Folgenden bereitgestellt ist:
einem Längsbereich, der sich von einem innenliegenden Ende (42in) des ersten Verbindungsteils (42) zu einem außenliegenden Ende (15ea) des Teils mit geringem Flächengewicht (15) erstreckt, und
einem Längsbereich, der sich von einem innenliegenden Ende (44in) des zweiten Verbindungsteils (44) zu einem anderen außenliegenden Ende (15eb) des Teils mit geringem Flächengewicht (15) erstreckt,
wobei der Teil mit hoher Steifigkeit ein Teil ist, der eine höhere Steifigkeit aufweist als die mittlere Position (FL).

2. Unterhosen-förmiger absorbierender Artikel nach Anspruch 1, wobei
der Teil mit hoher Steifigkeit einen komprimierten Teil (18) einschließt und
in einem Fall, in dem eine gedachte Linie (H) derart gezogen wird, dass eine Position eines längsgerichteten innenliegenden Endes (42ei, 44ei) von jedem Ende in der Breite (42e, 44e) von einem des ersten Verbindungsteils (42) und des zweiten Verbindungsteils (44) mit einem Mittelpunkt in der Breite (C1) verbunden ist, der sich an der mittleren Position (FL) in der Längsrichtung befindet,
sich der Teil mit geringem Flächengewicht (15) in der Längsrichtung zu einer Position erstreckt, die mit der gedachten Linie (H) identisch ist oder außenliegend in der Längsrichtung über die gedachte Linie (H) hinaus vorliegt, und
wobei der komprimierte Teil (18) in einem Teil bereitgestellt ist, der mit einem längsgerichteten Endteil (15e) des Teils mit geringem Flächengewicht (15) in einer Ansicht in der Dickenrichtung überlappt ist.

3. Unterhosen-förmiger absorbierender Artikel nach Anspruch 1 oder 2, wobei
der Teil mit hoher Steifigkeit einen komprimierten Teil (18) einschließt und
ein nicht-komprimierter Bereich (11X) in einem vorgegebenen Abschnitt bereitgestellt ist, der die mittlere Position (FL) in der Längsrichtung einschließt,
wobei der nicht-komprimierte Bereich (11X) ein Bereich ist, der nicht den komprimierten Teil (18) einschließt.

4. Unterhosen-förmiger absorbierender Artikel nach einem der Ansprüche 1 bis 3, wobei
der Teil mit hoher Steifigkeit in mindestens einem Teil eines Teils des Saugkörpers (11) bereitgestellt ist, der mit einem des ersten Verbindungsteils (42) und des zweiten Verbindungsteils (44) in einer Ansicht in der Dickenrichtung überlappt ist.

5. Unterhosen-förmiger absorbierender Artikel nach einem der Ansprüche 1 bis 3, wobei
das Haftmittel in dem ersten Verbindungsteil (42) und dem zweiten Verbindungsteil (44) sich entlang der Längsrichtung erstreckend an intermittierenden Stellen in der Breitenrichtung bereitgestellt ist und
der Teil mit hoher Steifigkeit in mindestens einem Teil eines Teils des Saugkörpers (11) bereitgestellt ist, der mit einem des ersten Verbindungsteils (42) und des zweiten Verbindungsteils (44) in einer Ansicht in der Dickenrichtung überlappt ist.

6. Unterhosen-förmiger absorbierender Artikel nach einem der Ansprüche 1 bis 5, wobei
der Teil mit hoher Steifigkeit einen komprimierten Teil (18) einschließt und
ein Überlappungsbereich (X2) und ein Nicht-Überlappungsbereich (X1) in Bezug auf die Längsrichtung bereitgestellt sind,
wobei der Überlappungsbereich (X2) ein Bereich ist, der den komprimierten Teil (18) einschließt, der mit dem Teil mit geringem Flächengewicht (15) überlappt ist,
der Nicht-Überlappungsbereich (X1) ein Bereich ist, der den komprimierten Teil (18) einschließt und der sich außenliegend von dem Überlappungsbereich (X2) befindet, und
wobei eine längsgerichtete Länge (LE2) des Überlappungsbereichs (X2) kleiner ist als oder gleich einer längsgerichteten Länge (LE1) des Nicht-Überlappungsbereichs (X1).

7. Unterhosen-förmiger absorbierender Artikel nach einem der Ansprüche 1 bis 6, wobei
der Teil mit hoher Steifigkeit einen komprimierten Teil (18) einschließt,
der Unterhosen-förmige absorbierende Artikel eine vertikale Richtung aufweist,
in der vertikalen Richtung in einem Zustand, in dem der absorbierende Hauptkörper (10) an der mittleren Position (FL) gefaltet wurde,
eine Seite, auf der der vordere Taillenteil (20) und der hintere Taillenteil (30) angeordnet sind, eine obere Seite ist und
eine Seite, die mit der mittleren Position (FL) übereinstimmt, eine untere Seite ist,
wobei ein Überlappungsbereich (X2) in Bezug auf die vertikale Richtung bereitgestellt ist,
wobei der Überlappungsbereich (X2) ein Bereich ist, der den komprimierten Teil (18) einschließt, der mit dem Teil mit geringem Flächengewicht (15) überlappt ist, und
wenn ein mittlerer Bereich des Teils mit geringem Flächengewicht (15ce) ein Bereich des Teils mit geringem Flächengewicht (15) ist, der sich in der vertikalen Richtung von einem unteren Ende des Überlappungsbereichs (X2) zu der mittleren Position (FL) erstreckt,
eine vertikale Länge (LE3) des mittleren Bereichs des Teils mit geringem Flächengewicht (15ce) länger ist als eine vertikale Länge (LE2) des Überlappungsbereichs (X2).

8. Unterhosen-förmiger absorbierender Artikel nach einem der Ansprüche 1 bis 7, wobei
der Teil mit hoher Steifigkeit einen komprimierten Teil (18) einschließt,
der Unterhosen-förmige absorbierende Artikel eine vertikale Richtung aufweist,
in der vertikalen Richtung in einem Zustand, in dem der absorbierende Hauptkörper (10) an der mittleren Position (FL) gefaltet wurde,
eine Seite, auf der der vordere Taillenteil (20) und der hintere Taillenteil (30) angeordnet sind, eine obere Seite ist und
eine Seite, die mit der mittleren Position (FL) übereinstimmt, eine untere Seite ist,
wobei ein Überlappungsbereich (X2) und ein Nicht-Überlappungsbereich (X1) in Bezug auf die vertikale Richtung bereitgestellt sind,
wobei der Überlappungsbereich (X2) ein Bereich ist, der den komprimierten Teil (18) einschließt, der mit dem Teil mit geringem Flächengewicht (15) überlappt ist,
der Nicht-Überlappungsbereich (X1) ein Bereich ist, der den komprimierten Teil (18) einschließt und der sich obenliegend von dem Überlappungsbereich (X2) befindet, und
wenn ein mittlerer Bereich des Teils mit geringem Flächengewicht (15ce) ein Bereich des Teils mit geringem Flächengewicht (15) ist, der sich in der vertikalen Richtung von einem unteren Ende des Überlappungsbereichs (X2) zu der mittleren Position (FL) erstreckt,
eine vertikale Länge (LE1) des Nicht-Überlappungsbereichs (X1) länger ist als eine vertikale Länge (LE3) des mittleren Bereichs des Teils mit geringem Flächengewicht (15ce).

9. Unterhosen-förmiger absorbierender Artikel nach einem der Ansprüche 1 bis 8, wobei
der Teil mit hoher Steifigkeit einen von Folgenden aufweist:
einen Teil, in dem ein Teil mit hoher Dichte in einer linearen Weise ausgebildet ist, und
einen Teil, in dem der Teil mit hoher Dichte punktförmig ist und eine Vielzahl der Teile mit hoher Dichte ausgebildet ist,
wobei der Teil mit hoher Dichte ein Teil ist, dessen Dichte höher ist als eine umgebende Fläche.

10. Unterhosen-förmiger absorbierender Artikel nach einem der Ansprüche 1 bis 9, wobei
der Unterhosen-förmige absorbierende Artikel eine vertikale Richtung aufweist,
in der vertikalen Richtung in einem Zustand, in dem der absorbierende Hauptkörper (10) an der mittleren Position (FL) gefaltet wurde,
eine Seite, auf der der vordere Taillenteil (20) und der hintere Taillenteil (30) angeordnet sind, eine obere Seite ist und
eine Seite, die mit der mittleren Position (FL) übereinstimmt, eine untere Seite ist, und
wenn eine Seite, auf der sich der vordere Taillenteil (20) befindet, eine vordere Seite ist und
wenn eine Seite, auf der sich der hintere Taillenteil (30) befindet, eine hintere Seite ist,
eine vertikale Länge des Teils mit geringem Flächengewicht (15) auf der vorderen Seite länger ist als eine vertikale Länge des Teils mit geringem Flächengewicht (15) auf der hinteren Seite.

11. Unterhosen-förmiger absorbierender Artikel nach einem der Ansprüche 1 bis 10, wobei
der Teil mit hoher Steifigkeit einen komprimierten Teil (18) einschließt,
ein Überlappungsbereich (X2) und ein Nicht-Überlappungsbereich (X1) bereitgestellt sind,
wobei der Überlappungsbereich (X2) ein Bereich ist, der den komprimierten Teil (18) einschließt, der mit dem Teil mit geringem Flächengewicht (15) in der Längsrichtung überlappt ist,
der Nicht-Überlappungsbereich (X1) ein Bereich ist, der den komprimierten Teil (18) einschließt und der sich außenliegend von dem Überlappungsbereich (X2) in der Längsrichtung befindet, und
wobei sich der Nicht-Überlappungsbereich (X1) bis zu einem eines längsgerichteten Endteils (11eL) und einem Endteil in der Breite (11eW) des Saugkörpers (11) erstreckt.

12. Unterhosen-förmiger absorbierender Artikel nach einem der Ansprüche 1 bis 10, wobei
der Unterhosen-förmige absorbierende Artikel weiter ein Paar von auslaufsicheren Wandteilen (50) umfasst, die jeweils auf Seiten in der Breite des absorbierenden Hauptkörpers (10) bereitgestellt sind,
das Paar von auslaufsicheren Wandteilen (50) jeweils Folgendes einschließt:
einen Grundendteil (55), der sich in der Dickenrichtung nicht aufstellen kann, und
einen aufstellenden Teil (50is), der sich von dem Grundendteil (55) zu einer Hautseite in der Dickenrichtung aufgrund eines elastischen Elements (52) aufstellen kann, das sich in der Längsrichtung dehnen und zusammenziehen kann,
wobei der Teil mit hoher Steifigkeit einen komprimierten Teil (18) einschließt und
in der Breitenrichtung der komprimierte Teil (18) in einer Gesamtheit eines Bereichs bereitgestellt ist, der sich von einem innenliegenden Ende (55is) von einem der Grundendteile (55) zu einem innenliegenden Ende (55is) von einem anderen der Grundendteile (55) erstreckt.

13. Unterhosen-förmiger absorbierender Artikel nach Anspruch 2, wobei
der komprimierte Teil (18) derart bereitgestellt ist, dass er zumindest einen Teil der gedachten Linie (H) in einer Draufsicht kreuzt.

14. Unterhosen-förmiger absorbierender Artikel nach einem der Ansprüche 1 bis 13, wobei
der Saugkörper (11) einen Saugkern (11A) und eine flüssigkeitsdurchlässige kernumhüllende Lage (11B) einschließt,
wobei die kernumhüllende Lage (11B) den Saugkern (11A) von einer Hautseite und einer Nicht-Hautseite in der Dickenrichtung abdeckt, und
wobei in dem Teil mit geringem Flächengewicht (15)
ein Teil der kernumhüllenden Lage (11B), der sich auf der Hautseite befindet, mit einem Teil der kernumhüllenden Lage (11B), der sich auf der Nicht-Hautseite befindet, verbunden ist.

## Revendications

1. Article absorbant en forme de culotte ayant une direction allant dans le sens longitudinal, une direction allant dans le sens de la largeur et une direction allant dans le sens de l'épaisseur qui se croisent les unes les autres dans un état non plié,
l'article absorbant en forme de culotte comportant :
une partie avant au niveau de la taille (20) ;
une partie arrière au niveau de la taille (30) ;
un corps principal absorbant (10) qui comprend un corps absorbant (11),
une première partie d'assemblage (42) dans laquelle une surface côté non orienté vers la peau d'une partie d'extrémité du corps principal absorbant (10) sur un côté dans la direction allant dans le sens longitudinal est assemblée au niveau de la partie avant au niveau de la taille (20) au moyen d'un adhésif ; et
une deuxième partie d'assemblage (44) dans laquelle une surface côté non orienté vers la peau d'une partie d'extrémité du corps principal absorbant (10) sur un autre côté dans la direction allant dans le sens longitudinal est assemblée au niveau de la partie arrière au niveau de la taille (30) au moyen d'un adhésif,
le corps principal absorbant (10) étant plié une fois au niveau d'une position centrale (FL) dans la direction allant dans le sens longitudinal,
des parties d'extrémité dans le sens de la largeur de la partie avant au niveau de la taille (20) et des parties d'extrémité dans le sens de la largeur de la partie arrière au niveau de la taille (30) étant assemblées au niveau d'une paire de parties d'assemblage au niveau de la taille,
le corps absorbant (11) comprenant une partie de faible masse surfacique (15) dans au moins une partie centrale dans la direction allant dans le sens longitudinal,
la partie de faible masse surfacique (15) s'étendant le long de la direction allant dans le sens longitudinal,
la partie de faible masse surfacique (15) ayant une masse surfacique inférieure par rapport à une zone environnante,
une partie de haute rigidité étant mise en oeuvre dans au moins l'une parmi
une région longitudinale s'étendant depuis une extrémité allant vers l'intérieur (42in) de la première partie d'assemblage (42) jusqu'à une extrémité allant vers l'extérieur (15ea) de la partie de faible masse surfacique (15) et
une région longitudinale s'étendant depuis une extrémité allant vers l'intérieur (44in) de la deuxième partie d'assemblage (44) jusqu'à une autre extrémité allant vers l'extérieur (15eb) de la partie de faible masse surfacique (15),
la partie de haute rigidité étant une partie qui a une rigidité supérieure par rapport à la position centrale (FL) .

2. Article absorbant en forme de culotte selon la revendication 1, dans lequel
la partie de haute rigidité comprend une partie comprimée (18), et
dans le cas de figure où une ligne virtuelle (H) est tracée de telle sorte qu'une position d'une extrémité longitudinale allant vers l'intérieur (42ei, 44ei) de chaque extrémité dans le sens de la largeur (42e, 44e) de l'une ou l'autre parmi la première partie d'assemblage (42) et la deuxième partie d'assemblage (44) est raccordée à un point central dans le sens de la largeur (C1) se trouvant au niveau de la position centrale (FL) dans la direction allant dans le sens longitudinal,
la partie de faible masse surfacique (15) s'étend dans la direction allant dans le sens longitudinal, jusqu'à une position identique à la ligne virtuelle (H) ou vers l'extérieur dans la direction allant dans le sens longitudinal au-delà de la ligne virtuelle (H), et
la partie comprimée (18) est mise en oeuvre dans une partie qui est chevauchée par une partie d'extrémité longitudinale (15e) de la partie de faible masse surfacique (15) dans une vue dans la direction allant dans le sens de l'épaisseur.

3. Article absorbant en forme de culotte selon la revendication 1 ou la revendication 2, dans lequel
la partie de haute rigidité comprend une partie comprimée (18), et
une région non comprimée (11X) est mise en oeuvre dans une plage prédéterminée qui comprend la position centrale (FL) dans la direction allant dans le sens longitudinal,
la région non comprimée (11X) étant une région qui ne comprend pas la partie comprimée (18).

4. Article absorbant en forme de culotte selon l'une quelconque des revendications 1 à 3, dans lequel
la partie de haute rigidité est mise en oeuvre dans au moins une partie d'une partie du corps absorbant (11) qui est chevauchée par l'une ou l'autre parmi la première partie d'assemblage (42) et la deuxième partie d'assemblage (44) dans une vue dans la direction allant dans le sens de l'épaisseur.

5. Article absorbant en forme de culotte selon l'une quelconque des revendications 1 à 3, dans lequel
l'adhésif dans la première partie d'assemblage (42) et dans la deuxième partie d'assemblage (44) est mis en oeuvre et va en s'étendant le long de la direction allant dans le sens longitudinal, au niveau d'emplacements intermittents dans la direction allant dans le sens de la largeur, et
la partie de haute rigidité est mise en oeuvre dans au moins une partie d'une partie du corps absorbant (11) qui est chevauchée par l'une ou l'autre parmi la première partie d'assemblage (42) et la deuxième partie d'assemblage (44) dans une vue dans la direction allant dans le sens de l'épaisseur.

6. Article absorbant en forme de culotte selon l'une quelconque des revendications 1 à 5, dans lequel
la partie de haute rigidité comprend une partie comprimée (18), et
une région de chevauchement (X2) et une région de non-chevauchement (X1) sont mises en oeuvre par rapport à la direction allant dans le sens longitudinal,
la région de chevauchement (X2) étant une région qui comprend la partie comprimée (18) qui est chevauchée par la partie de faible masse surfacique (15),
la région de non-chevauchement (X1) étant une région qui comprend la partie comprimée (18) et qui se trouve à l'extérieur de la région de chevauchement (X2), et
une longueur longitudinale (LE2) de la région de chevauchement (X2) est inférieure ou égale à une longueur longitudinale (LE1) de la région de non-chevauchement (X1).

7. Article absorbant en forme de culotte selon l'une quelconque des revendications 1 à 6, dans lequel
la partie de haute rigidité comprend une partie comprimée (18),
l'article absorbant en forme de culotte a une direction verticale,
dans la direction verticale, dans un état dans lequel le corps principal absorbant (10) a été plié au niveau de la position centrale (FL),
un côté sur lequel la partie avant au niveau de la taille (20) et la partie arrière au niveau de la taille (30) sont agencées est un côté supérieur, et
un côté correspondant à la position centrale (FL) est un côté inférieur,
une région de chevauchement (X2) est mise en oeuvre par rapport à la direction verticale,
la région de chevauchement (X2) étant une région qui comprend la partie comprimée (18) qui est chevauchée par la partie de faible masse surfacique (15), et
laisser une région centrale (15ce) de la partie de faible masse surfacique être une région de la partie de faible masse surfacique (15) qui s'étend dans la direction verticale depuis une extrémité inférieure de la région de chevauchement (X2) jusqu'à la position centrale (FL),
une longueur verticale (LE3) de la région centrale (15ce) de la partie de faible masse surfacique est plus longue qu'une longueur verticale (LE2) de la région de chevauchement (X2).

8. Article absorbant en forme de culotte selon l'une quelconque des revendications 1 à 7, dans lequel
la partie de haute rigidité comprend une partie comprimée (18),
l'article absorbant en forme de culotte a une direction verticale,
dans la direction verticale, dans un état dans lequel le corps principal absorbant (10) a été plié au niveau de la position centrale (FL),
un côté sur lequel la partie avant au niveau de la taille (20) et la partie arrière au niveau de la taille (30) sont agencées est un côté supérieur, et
un côté correspondant à la position centrale (FL) est un côté inférieur,
une région de chevauchement (X2) et une région de non-chevauchement (X1) sont mises en oeuvre par rapport à la direction verticale,
la région de chevauchement (X2) étant une région qui comprend la partie comprimée (18) qui est chevauchée par la partie de faible masse surfacique (15),
la région de non-chevauchement (X1) étant une région qui comprend la partie comprimée (18) et qui se trouve vers le haut par rapport à la région de chevauchement (X2), et
laisser une région centrale (15ce) de la partie de faible masse surfacique être une région de la partie de faible masse surfacique (15) qui s'étend dans la direction verticale depuis une extrémité inférieure de la région de chevauchement (X2) jusqu'à la position centrale (FL),
une longueur verticale (LE1) de la région de non-chevauchement (X1) est plus longue qu'une longueur verticale (LE3) de la région centrale (15ce) de la partie de faible masse surfacique.

9. Article absorbant en forme de culotte selon l'une quelconque des revendications 1 à 8, dans lequel
la partie de haute rigidité a l'une ou l'autre parmi
une partie dans laquelle une partie de haute densité est formée d'une manière linéaire, et
une partie dans laquelle la partie de haute densité est en forme de points et une pluralité des parties de haute densité sont formées,
la partie de haute densité étant une partie dont la densité est supérieure par rapport à une zone environnante.

10. Article absorbant en forme de culotte selon l'une quelconque des revendications 1 à 9, dans lequel
l'article absorbant en forme de culotte a une direction verticale,
dans la direction verticale, dans un état dans lequel le corps principal absorbant (10) a été plié au niveau de la position centrale (FL),
un côté sur lequel la partie avant au niveau de la taille (20) et la partie arrière au niveau de la taille (30) sont agencées est un côté supérieur, et
un côté correspondant à la position centrale (FL) est un côté inférieur, et
laisser un côté sur lequel la partie avant au niveau de la taille (20) se trouve être un côté avant, et
laisser un côté sur lequel la partie arrière au niveau de la taille (30) se trouve être un côté arrière,
une longueur verticale de la partie de faible masse surfacique (15) sur le côté avant est plus longue qu'une longueur verticale de la partie de faible masse surfacique (15) sur le côté arrière.

11. Article absorbant en forme de culotte selon l'une quelconque des revendications 1 à 10, dans lequel
la partie de haute rigidité comprend une partie comprimée (18),
une région de chevauchement (X2) et une région de non-chevauchement (X1) sont mises en oeuvre,
la région de chevauchement (X2) étant une région qui comprend la partie comprimée (18) qui est chevauchée par la partie de faible masse surfacique (15) dans la direction allant dans le sens longitudinal,
la région de non-chevauchement (X1) étant une région qui comprend la partie comprimée (18) et qui se trouve à l'extérieur de la région de chevauchement (X2) dans la direction allant dans le sens longitudinal, et
la région de non-chevauchement (X1) s'étend vers le haut jusqu'à l'une ou l'autre parmi une partie d'extrémité longitudinale (11eL) et une partie d'extrémité dans le sens de la largeur (11eW) du corps absorbant (11).

12. Article absorbant en forme de culotte selon l'une quelconque des revendications 1 à 10, dans lequel
l'article absorbant en forme de culotte comporte par ailleurs une paire de parties formant paroi anti-fuite (50) qui sont respectivement mises en oeuvre sur des côtés dans le sens de la largeur du corps principal absorbant (10),
la paire de parties formant paroi anti-fuite (50) comprennent chacune
une partie d'extrémité de base (55) qui ne peut pas s'élever dans la direction allant dans le sens de l'épaisseur, et
une partie surélevée (50is) qui peut s'élever depuis la partie d'extrémité de base (55) jusqu'à un côté orienté vers la peau dans la direction allant dans le sens de l'épaisseur en raison d'un élément élastique (52) qui peut s'étirer et se contracter dans la direction allant dans le sens longitudinal,
la partie de haute rigidité comprend une partie comprimée (18), et
dans la direction allant dans le sens de la largeur, la partie comprimée (18) est mise en oeuvre dans une totalité d'une région qui s'étend depuis une extrémité allant vers l'intérieur (55is) de l'une des parties d'extrémité de base (55) jusqu'à une extrémité allant vers l'intérieur (55is) d'une autre des parties d'extrémité de base (55) .

13. Article absorbant en forme de culotte selon la revendication 2, dans lequel
la partie comprimée (18) est mise en oeuvre de manière à croiser au moins une partie de la ligne virtuelle (H) dans une vue en plan.

14. Article absorbant en forme de culotte selon l'une quelconque des revendications 1 à 13, dans lequel
le corps absorbant (11) comprend une partie centrale absorbante (11A) et une feuille d'emballage de partie centrale perméable aux liquides (11B),
la feuille d'emballage de partie centrale (11B) recouvrant la partie centrale absorbante (11A) depuis un côté orienté vers la peau et un côté non orienté vers la peau dans la direction allant dans le sens de l'épaisseur, et
dans la partie de faible masse surfacique (15),
une partie de la feuille d'emballage de partie centrale (11B) se trouvant sur le côté orienté vers la peau est assemblée au niveau d'une partie de la feuille d'emballage de partie centrale (11B) se trouvant sur le côté non orienté vers la peau.
